# EUROPEAN PATENT SPECIFICATION

(11) **EP 4 094 754 B1**
(45) Date of publication and mention of the grant of the patent: **06.08.2025**
(21) Application number: 22186334.3
(22) Date of filing: 19.06.2019
(51) Int. Cl.: A61K 31/00, A61K 31/37, A61K 45/06, A61P 25/16, A61P 25/28, A61P 43/00

(54) **COMPOSITIONS FOR TREATING AND/OR PREVENTING PROTEIN-AGGREGATION DISEASES**
ZUSAMMENSETZUNGEN ZUR BEHANDLUNG UND/ODER PRÄVENTION VON PROTEINAGGREGATIONSERKRANKUNGEN
COMPOSITIONS POUR LE TRAITEMENT ET/OU LA PRÉVENTION DE MALADIES D'AGRÉGATION DES PROTÉINES

(30) Priority: 19.06.2018 EP 18382439
(43) Date of publication of application: 30.11.2022
(62) Divisional of application: 19730826.5
(73) Proprietor: Universidad Pablo de Olavide, 41013 Sevilla (ES)
(72) Inventor: MUÑOZ, Manuel J., 41013 Sevilla (ES); CARRIÓN, Ángel M., 41013 Sevilla (ES); PÉREZ-JIMÉNEZ, Mercedes M., 41013 Sevilla (ES)
(74) Representative: Hoffmann Eitle

(56) References cited:
- EP-A1- 1 193 250
- EP-A1- 3 011 950
- WO-A2-2016/090371
- US-A- 5 556 847
- US-A1- 2018 161 339
- YUE XING-HUA ET AL: "Steroid sulfatase inhibitor DU-14 protects spatial memory and synaptic plasticity from disruption by amyloid [beta] protein in male rats", HORMONES AND BEHAVIOR, ACADEMIC PRESS, NEW YORK, NY, US, vol. 83, 21 May 2016 (2016-05-21), pages 83 - 92, XP029617212, ISSN: 0018-506X, DOI: 10.1016/J.YHBEH.2016.05.019

## Description

### Technical field

The present invention is encompassed within the field of medicine and provides a composition for use in the treatments and/or prevention of protein-aggregation diseases.

### Background art

The proper functioning of organs and cells within an organism relies on the proper function of proteins. A protein is a biological entity that has a primary amino acid sequence; a secondary structure that forms protein domains and includes, most importantly, alpha helices and beta sheets; and a tertiary structure, a result of a complex folding of the peptide chain in three dimensions that involve the polypeptide chain backbone and amino acid side chain interactions. Some proteins work in a multi-subunit complex, where the arrangement of multiple proteins into a quaternary structure becomes crucial for their proper function.

The failure of proteins to fold into correct three-dimensional structures can lead to diseases called proteopathies (sometimes also referred to as protein-aggregation diseases, protein misfolding diseases, proteinopathies or protein conformational disorders). The failure may be due to one or more mutations in the proteins' gene or to environmental factors such as oxidative stress, alkalosis, acidosis, pH shift and osmotic shock. The misfolding of proteins can sometimes lead to clumping or aggregation into amyloid plaques or fibrils that can exacerbate a disease. Proteopathies cover a wide spectrum of afflictions, including neurodegenerative diseases (e.g., Alzheimer's, Parkinson's, polyglutamine diseases such as Huntingtin in Huntington's disease, prion diseases); amyloidosis of other non-nervous system proteins such as I1-antitrypsin, immunoglobulin light and heavy chains, lactadherin, apolipoprotein, gelsolin, lysozyme, fibrinogen, atrial natriuretic factor, keratin, lactoferrin and beta-2 microglobulin, among others); sickle cell disease; cataracts; cystic fibrosis; retinitis pigmentosa; and nephrogenic diabetes insipidus.

Amyloidosis refers to the pathological deposition of proteins in the form of congophilic, green birefringent fibrils, when congo red-stained, either dispersed or in the form of localized amyloidomas. Such deposits are symptomatic of several diseases, for example Alzheimer's Disease, inflammation-associated amyloid, type II diabetes, bovine spongiform encephalopathy (BSE), Creutzfeld-Jakob disease (CJD), scrapie and primary amyloidosis.

Amyloidoses are generally categorized into three groups: major systemic amyloidoses, major localized amyloidoses, and miscellaneous amyloidoses. Major systemic amyloidoses include: chronic inflammatory conditions (e.g., tuberculosis, osteomyelitis, etc.); non-infectious conditions such as juvenile rheumatoid arthritis, ankylosing spondylitis and Crohn's disease, etc.; familial Mediterranean Fever, plasma cell dyscrasia (primary amyloidosis) and various familial polyneuropathies and cardiomyopathies. Major localized amyloidoses include: dialysis-related amyloidosis, Alzheimer's disease, Down syndrome, Hereditary cerebral hemorrhage (Dutch), and non-traumatic cerebral hemorrhage of the elderly. Miscellaneous amyloidoses include: familial polyneuropathy (Iowa), familial amyloidosis (Finnish), hereditary cerebral hemorrhage (Icelandic), CJD, Medullary carcinoma of the thyroid, atrial amyloid, and diabetes mellitus (insulinomas). Other amyloidoses include those referenced in Louis W. Heck, "The Amyloid Diseases" in Cecil's Textbook of Medicine 1504-6 (W.B. Saunders & Co., Philadelphia, Pa.; 1996).

Transmissible spongiform encephalopathies which cause CJD and Gerstmann-Strässler-Scheinker (GSS) disease are described by B. Chesebro et al., "Transmissible Spongiform Encephalopathies: A Brief Introduction" in FIELD'S VIROLOGY 2845-49 (3rd Edition; Raven Publishers, Philadelphia, Pa.; 1996) and in D. C. Gajdusek, "Infectious amyloids: Subacute Spongiform Encephalopathies as Transmissible Cerebral Amyloidoses," 2851-2900 in FIELDS VIROLOGY (1996). Many of these diseases are likely mediated by prions, an infectious protein. See S. B. Prusineri, "Prions" in FIELDS VIROLOGY 2901-50 (1996) and the references contained therein.

Trying to eliminate specific fibrils has been the objective of significant research on amyloidosis but without success. Current treatment of amyloidosis involves chemotherapy agents or steroids, such as melphalan and dexamethasone. However, such a treatment is not appropriate for all patients and is not effective in many cases due to a lack of specificity (WO 2017/075540 A1). Similarly, the treatment of other proteopathies which are not necessarily associated with the formation of amyloids have also had limited success. Thus, there is a great need for alternatives that may safely and effectively treat or prevent proteopathies.

WO 2016/090371 discloses the use a compound that disrupts one Parkin ligase zinc finger for treating various disorders, such as cancer or neurodegenerative disorders. The steroid sulfatase inhibitor STX64 is cited amongst a list of potential compounds.

US 2018/161339 discloses the use of carbonic anhydrase inhibitors for inhibiting or reducing amyloid pathology or proteinopathy. The steroid sulfatase inhibitors Coumate and Emate are cited amongst a list of potential compounds.

The inventors of the present invention were initially interested in unravelling new elements that govern the genetic control of aging in order to improve our understanding of this intricate biological process. To this aim, they isolated thermotolerant mutants and identified an allele of the *sul-2* gene (a gene which encodes one of the three members of the *Caenorhabditis elegans* sulfatase family) which contained an inactivating point mutation. The inventors found that worms carrying the inactivated version of the *sul-2* gene lived longer than the wild-type.

Sulfatases are a large protein family involved in different biological processes and they affect a wide range of substrates. The collocation of *sul-2* in the sulfatase phylogenetic tree is uncertain. But, when compared to mammal sulfatases, *sul-2* clusters close to the type H, F, E, D Arylsulfatases and the type C steroid sulfatase that probably originate from a common ancestor gene. The inventors hypothesized that *sul-2* may exert its activity by modifying sulfated-steroid hormones.

Steroid hormone sulfatases are conserved proteins that participate in different processes, such as stimulating the proliferation of hormone-dependent cancers (Mueller et al., 2015. Endocr Rev. 36(5):526-63). Specific inhibitors for this type of enzyme have been generated, one of those is STX64 (Nussbaumer & Billich, 2004. Med Res Rev. 24(4):529-76). STX64 has been used to treat patients with hormone-dependent cancers (Stanway et al., 2006. Clin Cancer Res. 12(5):1585-92). The inventors treated wild type worms with STX64 and observed the same longevity effects as in the *sul-2* mutants. However, STX64 did not further increase the longevity of *sul-2* deletion mutants, indicating that the mechanism by which STX64 increases longevity is by inhibiting the sulfatase activity of SUL-2 (WO 2014/154927 A1).

### Figures

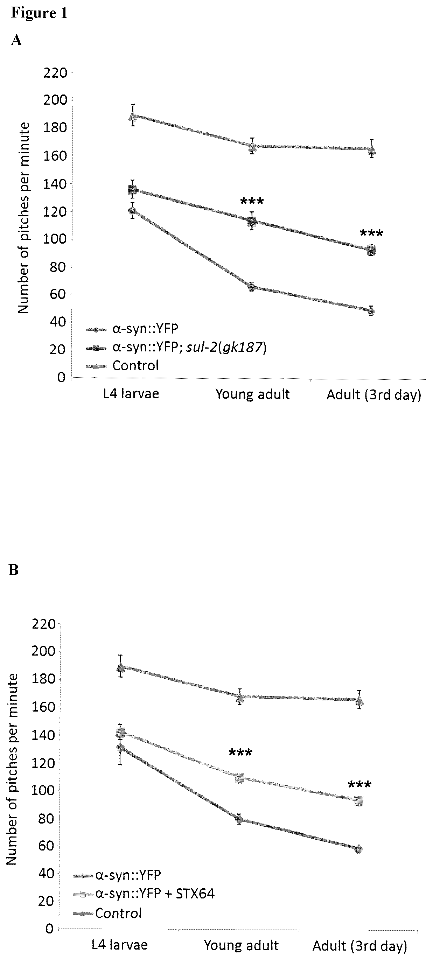

**Figure 1****:** Reduction of steroid hormone sulfatase (STS) activity ameliorates the symptoms of proteotoxicity in a C. *elegans* Parkinson's disease model strain NL5901 (Van Ham et al., 2010. Cell 142: 601-612). Nematodes expressing α-synuclein in muscle cells suffer from a reduced mobility as they age (measured as number of times per minute that the head of the worm crosses the longitudinal axis of the body (pitches)), but the rate is slowed down when (A) the nematode comprise the *sul-2(gkl87)* allele and (B) the nematodes are treated with STX64. n = 20; *** represents p<0.001.

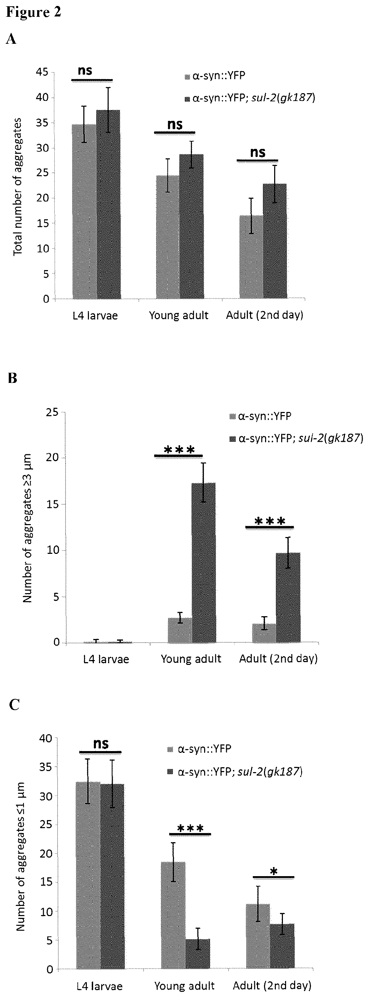

**Figure 2****:** Reduction of steroid hormone sulfatase (STS) activity alters the way that α-synuclein aggregates in a C. *elegans* Parkinson's disease model strain NL5901 (Van Ham et al., 2010. Cell 142: 601-612). The graphs show the (A) total number of aggregates, (B) number of aggregates ≥ 3 µm in size, and (C) number of aggregates ≤ 1 µm in size of nematodes expressing α-synuclein fused to YFP and nematodes expressing the same α-synuclein construct which additionally comprise an inactive *sul-*2 allele, i.e. *sul-2*(*gk187*). n = 20; *** represents p<0.001; * represents p<0.05.

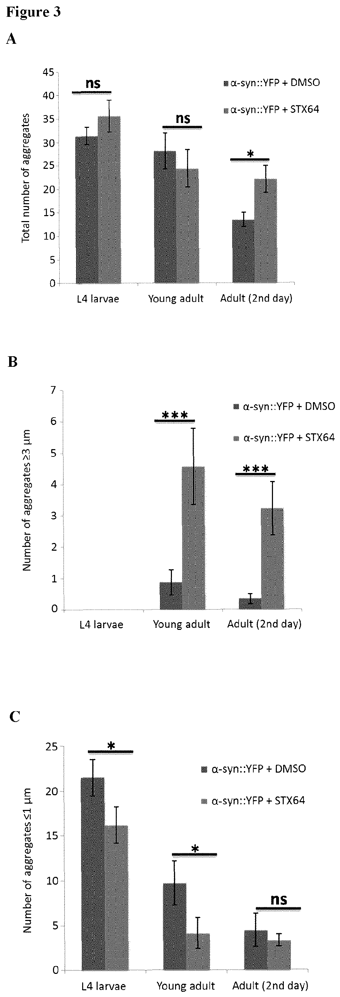

**Figure 3****:** Reduction of steroid hormone sulfatase (STS) activity alters the way that α-synuclein aggregates in a C. *elegans* Parkinson's disease model strain NL5901 (Van Ham et al., 2010. Cell 142: 601-612). The graphs show the (A) total number of aggregates, (B) number of aggregates ≥ 3 µm in size, and (C) number of aggregates ≤ 1 µm in size of nematodes expressing α-synuclein fused to YFP treated with DMSO and nematodes expressing the same α-synuclein construct which additionally have been treated with STX64. n = 20; *** means that p<0.001; * represents p<0.05.

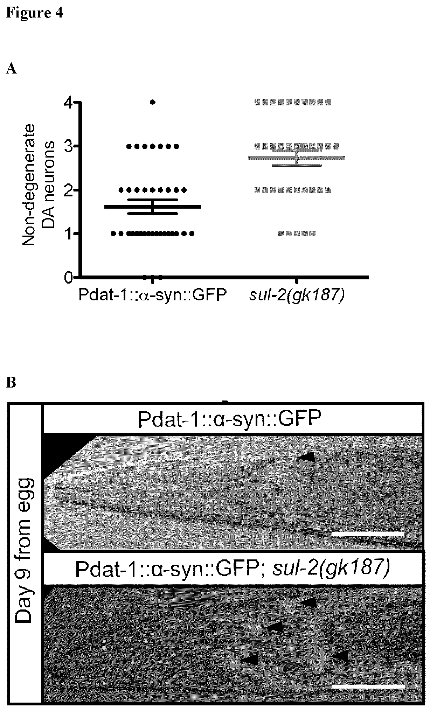

**Figure 4****:** Reduction of steroid hormone sulfatase (STS) activity ameliorates the symptoms of proteotoxicity in a C. *elegans* Parkinson's disease model strain UA44 (Cooper et al., 2006. Science 313: 324-328). (A) The survival of GFP-labeled dopaminergic neurons expressing human α-synuclein is increased when the nematode comprises the inactive *sul-2*(*gk187*) allele. The graph represents the number of living neurons in nematodes which are in day 6 when the nematode comprises the wild-type *sul-2* allele or the inactive *sul-2*(*gk187*) allele. (B) A representative image of a nematode which comprises the wild-type *sul-2* allele and a nematode which comprises the inactive *sul-2*(*gk187*) allele at day 9. Black arrows point towards living dopaminergic neurons. n= 37; p<0.0001.

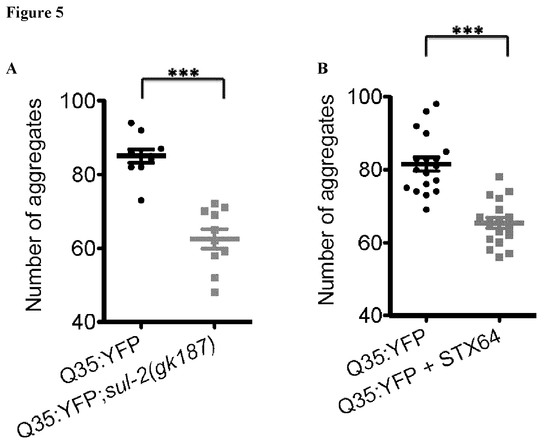

**Figure 5****:** Reduction of steroid hormone sulfatase (STS) activity ameliorates the symptoms of proteotoxicity in a C. *elegans* Huntington's disease model strain AM140 (Morley et al. 2002. PNAS. 99: 10417-10422). The nematodes have been modified to express a 35 polyglutamine repeat fused to YFP and the number of fluorescent aggregates was counted at day 5 of adulthood. (A) Graph shows a comparison between a nematode which comprises the wild-type allele of *sul-2* and a nematode which comprises the inactive mutant allele of *sul-2.* (B) Graph shows a comparison between a nematode which comprises the wild-type allele of *sul-2* and a nematode which comprises the wild-type allele of *sul-2* which was additionally treated with STX64. n = 20; *** represents p<0.001.

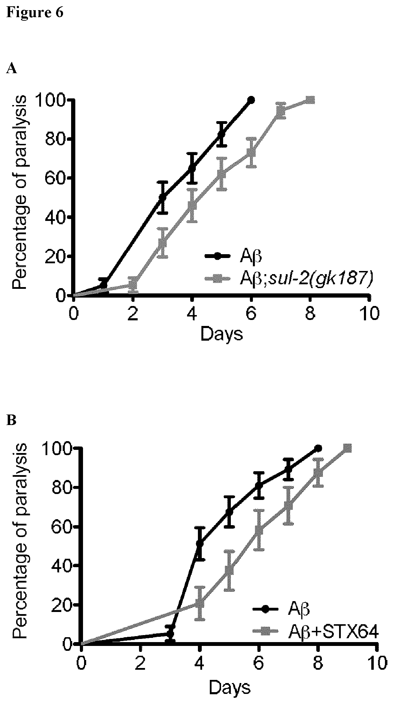

**Figure 6****:** Reduction of steroid hormone sulfatase (STS) activity ameliorates the symptoms of proteotoxicity in a C. *elegans* Alzheimer's disease model strain CL2006 (Link, 1995. PNAS. 92: 9368-9372). Nematodes expressing β-amyloid in muscle cells suffer from paralysis as they age, but the rate is slowed down when (A) the nematodes comprise the *sul-2*(*gk187*) allele and (B) the nematodes are treated with STX64. n = 50; p<0.001.

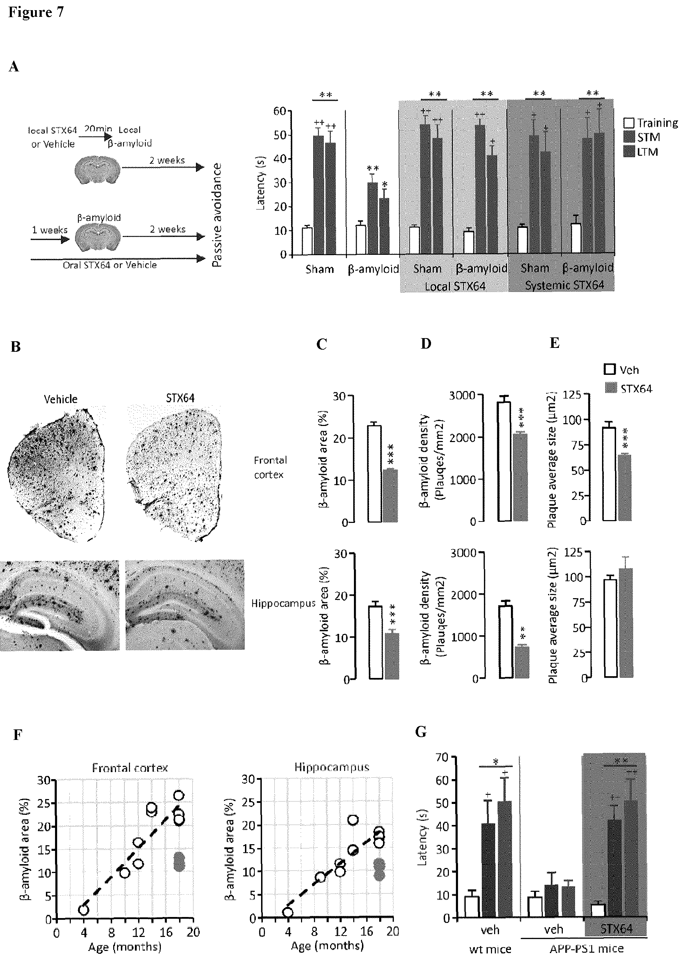

**Figure 7****:** STX64 treatment alleviated β-amyloid deposition and cognition deficiency in Alzheimer disease mouse models. (A) The effect of intrahippocampal and oral administration of STX64 in the passive avoidance test in wild type mice injected with β-amyloid oligomers in the hippocampus. The number of mice in each groups was >5. (B) Representative β-amyloid-immunoreactive images assessed in the frontal cortex and the hippocampus of APP-PS1 mice older than 15 months of age after 3-4 weeks of vehicle or STX64 intake (0.005mg/ml in drink water). (C)-(E), Quantification of percentage of β-amyloid area (C), deposition density (D) and average plaque size (E) in the frontal cortex and the hippocampus of >15 months-old APP-PS1 mice after 3-4 weeks of oral administration with STX64 or vehicle. n=4 mice per groups. (F) Time-course of β-amyloid deposition in APP-PS1 mice and the effect of 3-4 weeks STX64 oral treatment on β-amyloid area in the frontal cortex and the hippocampus. The number of microphotographs used was more than 3 per mouse. (G) Effect of oral administration with STX64 in more than 15 month-old APP-PS1 mice, and comparison with APP-PS1 and wild type mice older than 15 month in the passive avoidance test. The number of mice in each group was >5. In histological analysis, * represent statistically significant differences between vehicle and STX64 administrated APP-PS1 mice. In behavioral test, * represent statistically significant differences between the short-term and long-term memory sessions (STM and LTM, respectively) and the training session in the same experimental group; and, + represent statistically significant differences between the STM and LTM sessions between each experimental group and β-amyloid group. One symbol represents p<0.05; two repeats of the symbol represents p<0.01, and three two repeats of the symbol represents p<0.001.

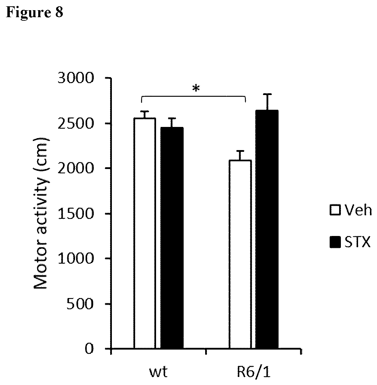

**Figure 8****:** STX64 prevented the deterioration of motor activity in a mouse model of Huntington's Disease. 1-month old R6/1 mice were treated daily with drinking water comprising STX64 or the vehicle per se. At the age of 2 months, the motor activity of the mice was tested. n >5 mice per group. Result represent mean±SEM. *, p<0,05.

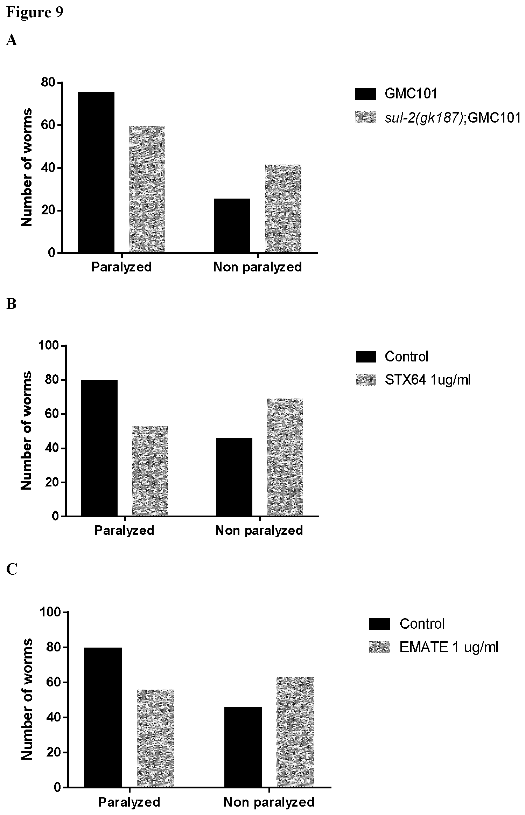

**Figure 9****:** A comparison between STX64 and EMATE in an Alzheimer's disease model, i.e. GMC101 strain of C. *elegans* (McColl et al., 2012. Mol Neurodegener. 7:57). (A) In the sul-2(gk187) deletion background the number of paralyzed worms was reduced. Graph shows the total number obtained from three biological replicate, n = 100 worms. Chi-square, 2-sided, p-value = 0.0161. (B) Treatment with STX64 at 1µg/ml reduced the number of paralyzed worms. Graph shows the total number obtained from three biological replicates, n > 120 worms. Chi-square, 2-sided, p-value = 0.0014. (C) Treatment with EMATE at 1µg/ml reduced the number of paralyzed worms. Graph shows the total number obtained from three biological replicates, n > 117 worms. Chi-square, 2-sided, p-value = 0.0091.

### Summary of the invention

Surprisingly, the inventors found that STX64 is able to treat or prevent the formation of oligomers and/or amyloids in a C. *elegans* Parkinson's model, C. *elegans* Huntington's model and C. *elegans* Alzheimer's disease model (see Figures 1-6). In Parikinson's disease, Huntington's disease and Alzheimer's disease the pathogenesis is driven by the production and/or deposition of protein aggregates (Murphy & Levine, 2010. J Alzheimers Dis. 19(1):311; Stefanis, 2012. Cold Spring Harb Perspect Med. 2(2): a009399; Daldin et al., 2017. Sci Rep. 7(1):5070). Thus, the treatment or prevention of aggregate species in these models renders it plausible that STX64 can be used to treat and/or prevent these protein-aggregation diseases. This was confirmed in a mouse model for Alzheimer's disease where it was found that STX64 decreased the formation of β-amyloid plaques and reverted cognitive deficiencies provoked by intrahippocampal administration of β-amyloid oligomers and in a transgenic mouse model of Alzheimer's idsease (see Figure 7).

Further, C. *elegans* comprising inactivation mutations in a sulfatase gene were similarly less prone to form harmful aggregates rendering it plausible that this effect is not only associated with STX64 but is applicable to any sulfatase inhibitor. The fact that this preventive/therapeutic effect was obtained in models for three different protein-aggregation diseases wherein the toxic proteins were expressed in muscle cells or neuron cells renders it plausible that a sulfatase inhibitor could be used to treat and/or prevent any protein-aggregation disease.

Thus, the present invention provides a composition comprising a steroid hormone sulfatase inhibitor for use in the treatment and/or prevention of a protein-aggregation disease, wherein amyloids and/or oligomers are removed, and/or their formation is prevented in the patient and/or animal, and wherein the formation of amyloids and/or oligomers is prevented in Parkinson's disease or Huntington's disease patients; or wherein the protein-aggregation disease is Parkinson's disease or Huntington's disease.

Further, the composition is a pharmaceutical composition comprising (i) the steroid hormone sulfatase inhibitor and (ii) pharmaceutically acceptable carrier and/or diluent.

### Detailed description of the invention

### Definitions

The terms *"individual", "patient"* or *"subject"* are used interchangeably in the present application to designate a human being and are not meant to be limiting in any way. The *"individual", "patient"* or *"subject"* can be of any age, sex and physical condition. The term *"animal",* as used in the present application, refers to any multicellular eukaryotic heterotroph which is not a human. In a preferred embodiment, the animal is selected from a group consisting of cats, dogs, pigs, ferrets, rabbits, gerbils, hamsters, guinea pigs, horses, worms, rats, mice, cows, sheep, goats, alpacas, camels, donkeys, llamas, yaks, giraffes, elephants, meerkats, lemurs, lions, tigers, kangaroos, koalas, bats, monkeys, chimpanzees, gorillas, bears, dugongs, manatees, seals and rhinoceroses.

As used herein, *"pharmaceutically acceptable carrier"* or *"pharmaceutically acceptable diluent"* means any and all solvents, dispersion media, coatings, antibacterial and antifungal agents, isotonic and absorption delaying agents, compatible with pharmaceutical administration. The term *"pharmaceutically acceptable excipient"* refers to any substance formulated alongside the active ingredient of a medication, included for the purpose of long-term stabilization, bulking up solid formulations that contain potent active ingredients in small amounts, or to confer a therapeutic enhancement on the active ingredient in the final dosage form, such as facilitating drug absorption, reducing viscosity, or enhancing solubility. Excipients can also be useful in the manufacturing process, to aid in the handling of the active substance concerned such as by facilitating powder flowability or non-stick properties, in addition to aiding in vitro stability such as prevention of denaturation or aggregation over the expected shelf life. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington: The Science and Practice of Pharmacy 22nd edition, Pharmaceutical press (2012), ISBN-13: 9780857110626 may also be included.

The terms *"treatment"* and *"therapy",* as used in the present application, refer to a set of hygienic, pharmacological, surgical and/or physical means used with the intent to cure and/or alleviate a disease and/or symptoms with the goal of remediating the health problem. The terms *"treatment"* and *"therapy"* include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

The term *"prevention",* as used in the present application, refers to a set of hygienic, pharmacological, surgical and/or physical means used to prevent the onset and/or development of a disease and/or symptoms. The term *"prevention"* encompasses prophylactic methods, since these are used to maintain the health of an animal or individual.

The term *"sulfatase inhibitor"* refers to any substance capable of reducing the activity of an enzyme of the esterase class that catalyzes the hydrolysis of sulfate esters. The substance may be a molecule that binds to any of the following elements: the gene that encodes the sulfatase enzyme, transcription factors of said gene, any of the expression products of said gene, for example, without being limited thereto, the messenger RNA or the sulfatase enzyme, and decreases or inhibits the expression and the activity of the molecule to which it binds, and/or its intracellular or extracellular signaling, thereby leading to total or partial inhibition of the activity of the sulfatase enzyme. The inhibitor may be selected from the list consisting of, without being limited thereto: antagonists against the sulfatase enzyme (preferably chemical), silencing RNA or specific antibody against the sulfatase enzyme (preferably, the antibody is monoclonal); in the present invention, this antibody may be defined as a neutralizing antibody against the effect of the sulfatase enzyme. Examples of chemical inhibitors of the activity of the sulfatase enzyme are, without being limited thereto, alternative substrates such as those in the series 2-(hydroxyphenyl) indol sulfate, synthetic or natural steroids which present inhibitory activity against STS, such as 5-androstene-3β, 17β-diol-3 sulfate, competitive inhibitors such as E1-MTP or EMATE, non-oestrogenic inhibitors such as DU-14 (CAS NO: 186303-55-9), COUMATE (4-methylcoumarin-7-O-sulphamate) or STX64 (i.e., compound of Formula (II)), or others, such as KW-2581 or STX213, whose IC₅₀ against the sulfatase enzyme has been determined in different studies (Purohit & Foster, 2012, J. Endocrinol., 212(2):99-110).

The term *"steroid hormone sulfatase"* ("*STS*") refers to any sulfatase enzyme involved in the metabolism of steroids. In particular, the enzymes catalyze the conversion of sulfated steroid precursors to the free steroid. An exemplary STS found in humans has been sequenced, characterized and the data have been deposited in the UniProtKB database under the accession number P08842. The term *"steroid hormone sulfatase inhibitor"* refers to any substance capable of reducing the activity of a steroid hormone sulfatase. The substance may be a molecule that binds to any of the following elements: the gene that encodes the STS enzyme, transcription factors of said gene, any of the expression products of said gene, for example, without being limited thereto, the messenger RNA or the STS enzyme, and decreases or inhibits the expression and the activity of the molecule to which it binds, and/or its intracellular signaling, thereby leading to total or partial inhibition of the activity of the STS enzyme. The inhibitor may be selected from the list consisting of, without being limited thereto: antagonists against the STS enzyme (preferably chemical), silencing RNA or specific antibody against the STS enzyme (preferably, the antibody is monoclonal); in the present invention, this antibody may be defined as a neutralising antibody against the effect of the STS enzyme. Examples of chemical inhibitors of the activity of the STS enzyme are, without being limited thereto, alternative substrates such as those in the series 2-(hydroxyphenyl) indol sulfate, synthetic or natural steroids which present inhibitory activity against STS, such as 5-androstene-3β, 17β-diol-3 sulfate, competitive inhibitors such as E1-MTP or EMATE, non-oestrogenic inhibitors such as DU-14, COUMATE (4-methylcoumarin-7-O-sulphamate) or STX64 (i.e., compound of Formula (II)), or others, such as KW-2581 or STX213, whose IC₅₀ against the sulfatase enzyme has been determined in different studies (Purohit & Foster, 2012, J. Endocrinol., 212(2):99-110).

The terms *"protein-aggregation disease", "proteopathy", "proteinopathy"* or *"protein misfolding diseases"* refers to any disease in which certain proteins become structurally abnormal and thereby disrupt the function of cells, tissues and organs of the body. Often the proteins fail to fold into their normal configuration; in this misfolded state, the proteins can become toxic in some way or they can lose their normal function. Non-limiting examples of protein-aggregation diseases include systemic AL amyloidosis, Alzheimer's Disease, Diabetes mellitus type 2, Parkinson's disease, Transmissible spongiform encephalopathy e.g. Bovine spongiform encephalopathy, Fatal Familial Insomnia, Huntington's Disease, Medullary carcinoma of the thyroid, Cardiac arrhythmias, Atherosclerosis, Rheumatoid arthritis, Aortic medial amyloid, Prolactinomas, Familial amyloid polyneuropathy, Hereditary non-neuropathic systemic amyloidosis, Dialysis related amyloidosis, Finnish amyloidosis, Lattice corneal dystrophy, Cerebral amyloid angiopathy, Cerebral amyloid angiopathy (Icelandic type), Sporadic Inclusion Body Myositis, Amyotrophic lateral sclerosis (ALS), Prion-related or Spongiform encephalopathies, such as Creutzfeld-Jacob, Dementia with Lewy bodies, Frontotemporal dementia with Parkinsonism, Spinocerebellar ataxias, Spinocerebellar ataxia, Spinal and bulbar muscular atrophy, Hereditary dentatorubral-pallidoluysian atrophy, Familial British dementia, Familial Danish dementia, Non- neuropathic localized diseases, such as in Type II diabetes mellitus, Medullary carcinoma of the thyroid, Atrial amyloidosis, Hereditary cerebral haemorrhage with amyloidosis, Pituitary prolactinoma, Injection-localized amyloidosis, Aortic medial amyloidosis, Hereditary lattice corneal dystrophy, Corneal amyloidosis associated with trichiasis, Cataract, Calcifying epithelial odontogenic tumors, Pulmonary alveolar proteinosis, Inclusion-body myositis, Cutaneous lichen amyloidosis, and Non-neuropathic systemic amyloidosis, such as AL amyloidosis, AA amyloidosis, Familial Mediterranean fever, Senile systemic amyloidosis, Familial amyloidotic polyneuropathy, Hemodialysis-related amyloidosis, ApoAI amyloidosis, ApoAII amyloidosis, ApoAIV amyloidosis, Finnish hereditary amyloidosis, Lysozyme amyloidosis, Fibrinogen amyloidosis, Icelandic hereditary cerebral amyloid angiopathy, familial amyloidosis, and systemic amyloidosis which occurs in multiple tissues, such as light-chain amyloidosis, and other various neurodegenerative disorders.

The term *"protein aggregate"* refers to any accumulation of abnormally folded proteins which cause and/or are associated with the negative progression of a protein-aggregation disease.

The term *"amyloid"* refers to a form of protein aggregates wherein the aggregates form unbranched fibers that bind Congo Red and then show green birefringence when viewed between crossed polarizers (for example, see Eisenberg & Jucker, 2012. Cell. 148(6):1188-203 and Sipe et al., 2012. Amyloid. 19(4):167-70).

The term *"oligomer"* refers to any accumulation of abnormally folded proteins which cause and/or are associated with the negative progression of a protein-aggregation disease and does not satisfy the definition of an amyloid. For example, polyglutamine oligomers cause and/or are associated with the negative progression of Huntington's disease (see Hoffner & Dijan, 2014. Brain Sci. 4(1): 91-122).

### Composition

The present invention provides a composition comprising a steroid hormone sulfatase inhibitor for use in the treatment and/or prevention of a protein-aggregation disease, wherein amyloids and/or oligomers are removed, and/or their formation is prevented in the patient and/or animal, and wherein the formation of amyloids and/or oligomers is prevented in Parkinson's disease or Huntington's disease patients; or wherein the protein-aggregation disease is Parkinson's disease or Huntington's disease.

Further, the composition is a pharmaceutical composition comprising (i) the steroid hormone sulfatase inhibitor and (ii) pharmaceutically acceptable carrier and/or diluent.

Preferred embodiments for the kits and compositions of the present invention are provided below.

### Sulfatase inhibitor

The sulfatase inhibitor is a steroid hormone sulfatase inhibitor. Preferably, the steroid hormone sulfatase inhibitor is selected from the list consisting of 2-(hydroxyphenyl) indol sulfate, DU-14, 5-androstene-3β, 17β-diol-3 sulfate, E1-MTP, EMATE, COUMATE, STX64, KW-2581, STX213, morpholine, silencing RNA and specific antibody against the STS enzyme.

In a preferred embodiment, the sulfatase inhibitor or steroid hormone sulfatase inhibitor is a compound of Formula (I): 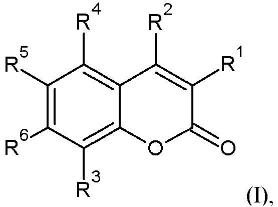 wherein:
(a) R₁-R₆ are independently selected from hydrogen, halogen (fluorine, chlorine, bromine, iodine or astatine), hydroxyl, sulfamate (OSO₂NH₂), alkyl and salts thereof;
(b) at least one of R₁-R₆ is a sulfamate group; and
two or more of R₁-R₆ are linked together to form an additional cyclic structure.

In a preferred embodiment, R₁ and R₂ form an additional cyclic structure comprising 3-10 carbons. In a preferred embodiment, R₆ is OSO₂NH₂. In a preferred embodiment, the alkyl is a C₁-C₆ alkyl.

In a preferred embodiment, the sulfatase inhibitor is the compound of Formula (II):

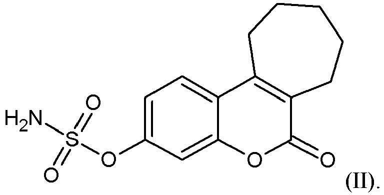

Methods of synthesizing the above compounds of Formula (I) and (II) have been disclosed in WO 97/30041.

### Protein-aggregation disease

Protein aggregates such as amyloids and oligomers have been associated with a number of diseases. In some cases, these protein aggregates can become toxic and can cause significant damage to cells and tissue. This toxicity is thought to be one of the contributing factors causing and/or contributing to the pathology of protein-aggregation diseases.

Further, the abnormal processing and folding of a protein linked to a protein-aggregation disease can start decades before the outward symptoms of the protein-aggregation disease can be observed (Jack et al., 2010. Lancet Neurol. 9(1):119-28). Thus, in a preferred embodiment, amyloids and/or oligomers are removed and/or their formation is prevented in the patient and/or animal as a result of administering any one of the compositions of the present invention. Further, in a preferred embodiment, the sulfatase inhibitor treats and/or prevents proteotoxicity in a protein-aggregation disease. The term *"proteotoxicity"* refers to any impairment of cell function caused by misfolding of a protein.

By directly targeting the formation of protein aggregates, the compositions and kits of the present invention are able to treat and/or prevent a protein-aggregation disease in patients and/or animals who are at the early stages of a protein-aggregation disease but still do not show any outward symptoms. Further, the compositions and kits of the present invention also treat the advanced stages of a protein-aggregation disease as demonstrated in Example 2 and Figures 7F and 7G.

In a preferred embodiment, the patient and/or animal have undergone the pathophysiological changes that cause protein aggregation but have not yet reached the stage of the disease where outward symptoms are observable. In other words, the patient and/or animal is at an early stage of the disease. The term *"outward symptom"* refers to any symptom which can be observed by a physician using any non-invasive procedure.

In a preferred embodiment, the sulfatase inhibitor slows down the progression of a protein-aggregation disease by inhibiting the formation of protein aggregates and/or the sulfatase inhibitor delays the onset of a protein-aggregation disease by inhibiting the formation of protein aggregates.

Protein-aggregation disease is selected from a list consisting of systemic AL amyloidosis, Alzheimer's Disease, Diabetes mellitus type 2, Parkinson's disease, Transmissible spongiform encephalopathy e.g. Bovine spongiform encephalopathy, Fatal Familial Insomnia, Huntington's Disease, Medullary carcinoma of the thyroid, Cardiac arrhythmias, Atherosclerosis, Rheumatoid arthritis, Aortic medial amyloid, Prolactinomas, Familial amyloid polyneuropathy, Hereditary non-neuropathic systemic amyloidosis, Dialysis related amyloidosis, Finnish amyloidosis, Lattice corneal dystrophy, Cerebral amyloid angiopathy, Cerebral amyloid angiopathy (Icelandic type), Sporadic Inclusion Body Myositis, Amyotrophic lateral sclerosis (ALS), Prion-related or Spongiform encephalopathies, such as Creutzfeld-Jacob, Dementia with Lewy bodies, Frontotemporal dementia with Parkinsonism, Spinocerebellar ataxias, Spinocerebellar ataxia, Spinal and bulbar muscular atrophy, Hereditary dentatorubral-pallidoluysian atrophy, Familial British dementia, Familial Danish dementia, Non- neuropathic localized diseases, such as in Type II diabetes mellitus, Medullary carcinoma of the thyroid, Atrial amyloidosis, Hereditary cerebral haemorrhage with amyloidosis, Pituitary prolactinoma, Injection-localized amyloidosis, Aortic medial amyloidosis, Hereditary lattice corneal dystrophy, Corneal amyloidosis associated with trichiasis, Cataract, Calcifying epithelial odontogenic tumors, Pulmonary alveolar proteinosis, Inclusion-body myositis, Cutaneous lichen amyloidosis, and Non-neuropathic systemic amyloidosis, such as AL amyloidosis, AA amyloidosis, Familial Mediterranean fever, Senile systemic amyloidosis, Familial amyloidotic polyneuropathy, Hemodialysis-related amyloidosis, ApoAI amyloidosis, ApoAII amyloidosis, ApoAIV amyloidosis, Finnish hereditary amyloidosis, Lysozyme amyloidosis, Fibrinogen amyloidosis, Icelandic hereditary cerebral amyloid angiopathy, familial amyloidosis, and systemic amyloidosis which occurs in multiple tissues, such as light-chain amyloidosis, and other various neurodegenerative disorders. Preferably, the protein-aggregation disease is selected from a list consisting of Alzheimer's disease, Parkinson's disease and Huntington's disease. In a preferred embodiment, the protein-aggregation disease is not Alzheimer's disease and/or a type of cancer.

According to the present invention, the protein-aggregation disease is selected from Parkinson's disease and Huntington's disease. In a preferred embodiment, amyloids and/or oligomers are removed and/or their formation is prevented in Parkinson's disease or Huntington's disease patients.

In a preferred embodiment, the protein-aggregation disease is a central nervous system localized protein-aggregation disease. In a preferred embodiment, the protein-aggregation disease is also a neurodegenerative disease. The term *"neurodegenerative disease"* refers to any disorder characterized by the progressive loss of structure or function of neurons, including death of neurons. For example, Alzheimer's disease is an example or a protein-aggregation disease and an example of a neurodegenerative disease. However, only the treatment of Parkinson's disease and Huntington's disease forms part of the present invention.

### Combined embodiments of sulfatase inhibitors and protein-aggregation diseases

In a preferred embodiment, the sulfatase inhibitor is selected from the list consisting of 2-(hydroxyphenyl) indol sulfate, 5-androstene-3β, DU-14, 17β-diol-3 sulfate, E1-MTP, EMATE, COUMATE, STX64, KW-2581, STX213, morpholine, silencing RNA and specific antibody against the STS enzyme; or the sulfatase inhibitor is a compound of Formula (I): 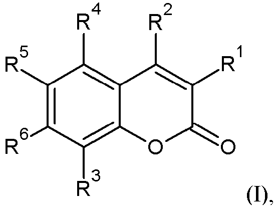 wherein:
(a) R₁-R₆ are independently selected from hydrogen, halogen (fluorine, chlorine, bromine, iodine or astatine), hydroxyl, sulfamate (OSO₂NH₂), alkyl and salts thereof;
(b) at least one of R₁-R₆ is a sulfamate group; and

two or more of R₁-R₆ are linked together to form an additional cyclic structure; and
the protein-aggregation disease is selected from Huntington's disease and Parkinson's disease.

In a preferred embodiment, the sulfatase inhibitor is STX64 (i.e., the compound of Formula (II)) and the protein-aggregation disease is selected from Huntington's disease and Parkinson's disease. In a preferred embodiment, the sulfatase inhibitor is STX64 and the protein-aggregation disease is Huntington's disease. In a preferred embodiment, the sulfatase inhibitor is STX64 and the protein-aggregation disease is Parkinson's disease.

### Pharmaceutical compositions

In a preferred embodiment, the composition is a pharmaceutical composition further comprising a pharmaceutically acceptable carrier and/or diluent. Preferably, the pharmaceutical composition may further comprise a pharmaceutically acceptable excipient.

A pharmaceutical composition as described herein may also contain other substances. These substances include, but are not limited to, cryoprotectants, lyoprotectants, surfactants, bulking agents, anti-oxidants, and stabilizing agents. In some embodiments, the pharmaceutical composition may be lyophilized.

The term *"cryoprotectant"* as used herein, includes agents which provide stability to the compositions against freezing-induced stresses. Cryoprotectants may also offer protection during primary and secondary drying and long-term product storage. Non-limiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In one embodiment, a lyoprotectant is added to a pharmaceutical composition described herein. The term *"lyoprotectant"* as used herein, includes agents that provide stability to the compositions during the freeze-drying or dehydration process (primary and secondary freeze- drying cycles. This helps to minimize product degradation during the lyophilization cycle, and improve the long-term product stability. Non-limiting examples of lyoprotectants include sugars, such as sucrose or trehalose; an amino acid, such as monosodium glutamate, non-crystalline glycine or histidine; a methylamine, such as betaine; a lyotropic salt, such as magnesium sulfate; a polyol, such as trihydric or higher sugar alcohols, e.g., glycerin, erythritol, glycerol, arabitol, xylitol, sorbitol, and mannitol; propylene glycol; polyethylene glycol; pluronics; and combinations thereof. The amount of lyoprotectant added to a pharmaceutical composition is generally an amount that does not lead to an unacceptable amount of degradation when the pharmaceutical composition is lyophilized.

In some embodiments, a bulking agent is included in the pharmaceutical composition. The term *"bulking agent"* as used herein, includes agents that provide the structure of the freeze- dried product without interacting directly with the pharmaceutical product. In addition to providing a pharmaceutically elegant cake, bulking agents may also impart useful qualities in regard to modifying the collapse temperature, providing freeze-thaw protection, and enhancing the stability over long-term storage. Non-limiting examples of bulking agents include mannitol, glycine, lactose, and sucrose. Bulking agents may be crystalline (such as glycine, mannitol, or sodium chloride) or amorphous (such as dextran, hydroxyethyl starch) and are generally used in formulations in an amount from 0.5% to 10%.

Other pharmaceutically acceptable carriers, excipients, or stabilizers, such as those described in Remington's Pharmaceutical Sciences 16th edition, Osol, A. Ed. (1980) or Remington: The Science and Practice of Pharmacy 22nd edition, Pharmaceutical press (2012), ISBN-13: 9780857110626 may also be included in a pharmaceutical composition described herein, provided that they do not adversely affect the desired characteristics of the pharmaceutical composition.

For solid pharmaceutical compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For solution for injection, the pharmaceutical composition may further comprise cryoprotectants, lyoprotectants, surfactants, bulking agents, anti-oxidants, stabilizing agents and pharmaceutically acceptable carriers. For aerosol administration, the pharmaceutical compositions are generally supplied in finely divided form along with a surfactant and propellant. The surfactant must, of course, be nontoxic, and is generally soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, as with, e.g., lecithin for intranasal delivery. For suppositories, traditional binders and carriers may include, for example, polyalkalene glycols or triglycerides.

In a preferred embodiment, the composition the present invention is prepared for oral, sublingual, buccal, intranasal, intravenous, intramuscular, intraperitoneal and/or inhalation-mediated administration.

### Administration

The composition of the present invention may be administered using any route known to the skilled person. In a preferred embodiment, the composition of the present invention is administered transdermally, sublingually, intravenously, intranasally, intracerebroventricularly, intraarterially, intracerebrally, intramuscularly, intraperitoneally, orally or via inhalation.

In a preferred embodiment, the composition of the present invention is administered transdermally, sublingually, intravenously, intraperitoneally, orally or via inhalation. Where the composition is administered via inhalation, the composition may be aerosolized and administered via, for example, an anesthesia mask.

In a preferred embodiment, the composition of the present invention is administered transdermally, sublingually, intravenously, subcutaneously, orally or via inhalation. Preferably, the composition is administered orally or sublingually.

In a preferred embodiment, the composition comprises a therapeutically effective amount of sulfatase inhibitor. The term *"therapeutically effective amount"* refers to an amount of sulfatase inhibitor in a composition which has a therapeutic effect and which is able to treat and/or prevent a protein-aggregation disease. In a preferred embodiment, a dose of 0.01 to 100 mg/kg of the sulfatase inhibitor is administered to the patient. Preferably, a dose of 0.01 to 10 mg/kg is administered to the patient. More preferably, a dose of 0.05 to 1 mg/kg is administered to the patient.

In a preferred embodiment, the composition is used in a combination therapy with any other treatment or therapy commonly used to treat and/or prevent a protein-aggregation disease. In a preferred embodiment, the composition is used in a combination therapy with donepezil, rivastigmine, galantamine, memantine, levodopa, carbidopa and/or tetrabenazine.

The compositions of the present invention may be administered once or more than once. A skilled person will be able to ascertain the most effective dosage regimen for the patient. For example, the most effective dosage regimen may be one where the patient is administered the composition twice daily, once a day, once every three days, once a week, once a month, once every three months, once every six months or once every year.

### Examples

*C*. elegans containing a *sul-2*(*gk187*) knock-out allele (WormBase ID: WBVar00145594) were generated by the C. *elegans* Gene Knockout Consortium (The C. elegans Deletion Mutant Consortium, 2012. G3: GENES, GENOMES, GENETICS. 2(11):1415-1425) and deposited at the Caenorhabidits Genetic Center (https://cbs.umn.edu/cgc/home). The *sul-2*(*gk187*) allele consists of a deletion of 131 amino acids that includes the catalytic core of the sulfatase. The deletion generates a frame shift and only the first four amino acids of the original sequence are conserved. Therefore, we consider *sul-2*(*gk187*) a null mutant allele of *sul-2.* The mutant lesion is available at wormbase (http://www.wormbase .org/species/c elegans/variation/WBVar00145594#02-45-3).

### Example 1: Effects of STX64 on nematode models of neurodegenerative diseases

***C. elegans* models of Parkinson's disease:** We tested if *sul-2* mutations or STX64 (Sigma cat. No. S1950) treatment at 1 µg/ml concentration dissolved in DMSO improved the symptoms caused by the overexpression of human α-synuclein in muscle cells (see the following references for information on the model used: van Ham et al., 2008. PLoS Genet. 4(3):e1000027; Gidalevitz et al., 2009. PLoS Genet. 5(3):e1000399; van Ham et al., 2010. Cell. 142(4):601-12). In particular, nematodes were synchronized at 20 °C and monitored every two days. On each day of monitoring, a nematode was placed in a drop of M9 buffer, allowing 30 seconds accommodation and later counting number of pitches in one minute, assuming that a pitch occurs when the head of the nematode crosses the axial axis. N=20 for each day assayed and condition (this protocol was adapted from Van Ham et al., 2010. Cell. 142: 601-612). As one can see in Figure 1A and 1B, *sul-2* mutation or treatment with STX64 significantly improved mobility.

Further, we tested the effect of the *sul-2* deletion and the administration of STX64 on the aggregation of α-synuclein. To quantify the aggregates, animals were transferred to pads comprising 2% agarose and immobilized using 25 mM levamisole. The number of α-synuclein aggregates was determined by counting fluorescent aggregates at 40x magnification, from first bulb pharyngeal to second bulb. Loss of function or inhibition of SUL-2 decreased the number of smaller aggregates while increasing the number of bigger aggregates of α-synuclein (see Figures 2 and 3), suggesting a better handling of protein aggregates in animals with reduced STS activity (Roberts & Brown, 2015. Biomolecules. 5(2):282-305; Moll et al., 2016. FASEB J. 30(4):1656-69).

In an alternative C. *elegans* model of Parkinson's which expresses α-synuclein in GFP-labeled dopaminergic neurons, it was found that the dopaminergic neurons die due to α-synuclein toxicity (Cooper et al., 2006. Science. 313(5785):324-8). Consistent with the previous findings, *sul-2* mutants showed increased neuron survival (see Figure 4), indicating a neuroprotective action of reducing STS activity.

***C. elegans* model of Huntington's disease:** In a Huntington's disease model expressing 35 polyglutamine (polyQ) repeats fused to a fluorescent protein, we found that both the *sul-2* mutation and the administration of STX64 at 1 µg/ml concentration dissolved in DMSO reduced the total number of aggregates (see Figure 5). To quantify the aggregates, animals were transferred to pads comprising 2% agarose and immobilized using 25 mM levamisole. The number of polyQ aggregates was determined by counting fluorescent aggregates at 10x magnification, from first bulb pharyngeal to second bulb.

***C. elegans* model of Alzheimer's disease:** We also tested an Alzheimer's disease model expressing β-amyloid protein in muscle cells which causes paralysis in the nematode as it ages. Nematodes were synchronized at 20 °C. Paralysis was monitored from 1st day of adulthood, by observing whether there is a lack of movement after the nematode was stimulated with a platinum pick. In addition, to track paralyzed nematodes, we visualized which nematodes had a bacteria halo around their head because paralysis prevents displacement of food. Consistent with our previous data, the *sul-2* mutation and STX64 treatment delayed paralysis (see Figure 6).

### Example 2: Effects of STX64 on mammalian models of neurodegenerative diseases (Reference example)

**Mice strains and conditions:** The male Swiss (CD1) and APP-PS1 (Blanchard et al., 2003. Exp Neurol. 184(1):247-63) mice used in this study were purchased from an authorized provider (University of Seville, Spain) and they were habituated to standard animals housing conditions for 2-3 weeks (a 12 h light/dark cycle, temperature and humidity). Behavioral studies were performed with 8 week-old Swiss mice, and >15 month-old APP-PS1 mice in C57Black background. For histological studies, male APP-PS 1 mice from 2 to > 15 months of age were used. All experiments were performed in accordance with European Union guidelines (2010/63/EU) and with Spanish regulations for the use of laboratory animals in chronic experiments (RD 53/2013 on the care of experimental animals: BOE 08/02/2013), and the approval of the University Pablo de Olavide animal care committees was obtained prior to performing this study.

**Mice local drug infusion:** Mice were anesthetized with 4% chloral hydrate (10 µL/kg of body weight, i.p.) and when fully anesthetized, they were situated in a stereotactic frame. In order to injure the hippocampus, 0.5µl of 5µM solution of β-amyloid oligomers were injected bilaterally into the dorsal hippocampus of the mice at the following stereotactic coordinates: AP = -2.2 mm, ML = ±1.5 mm, V = -1.5 mm from the Bregma. The mice were then allowed to recover for at least 15 days. Those mice that also received STX64 were administered 0.5 µl of a 1 mg/ml solution of STX64 20 minutes before β-amyloid oligomers were administrated. STX64 was infused in the same rostral hippocampus coordinates. STX64 and β-amyloid oligomers were delivered at a rate of 0.2 µl/min through an injection syringe (Hamilton), and left in place for 2.5 min following infusion.

**Mice Oral STX administration:** STX64 was dissolved in drinking water at 0.005mg/ml. Mice were exposed to STX solution during 3-4 weeks and the water intake was registered every day during the treatment. The estimated daily dose of STX64 was between 1-2 mg/Kg of body weight.

**Step-through passive avoidance (PA) test:** Mice have an innate preference for dark and enclosed environments. During the habituation phase, mice were handled and allowed to move freely for 1 minute in a chamber (47×18×26 cm, manufactured by Ugo Basile) that is divided symmetrically into one light and one dark compartment (each measuring 28.5 cm×18 cm×26 cm). During the training phase, mice were briefly confined to the light compartment and then 30 seconds later, the door separating the dark-light compartments was opened. Once mice entered the dark compartment, the door was closed automatically and the mice received an electrical stimulation (0.5mA, 5s and 0.3mA, 5s for Swiss and C57Black mice, respectively) delivered through the metal floor. In the retention tests performed at the times indicated, mice that recalled the electrical shock experience when replaced in the light compartment avoided, or at least took longer, to enter the dark compartment. Thus, the latency to enter into the dark compartment (escape latency) is a measure of information learning or memory retention depending on how long after the training session the test was carried out. Escape latency (s), the training, short- and long-term memory (STM and LTM, respectively) sessions are represented.

**Immunohistochemistry and histological analysis:** For immunohistochemistry, an antibody against β-amyloid (1:3000, Sigma-Aldrich) was used. Antibody staining was visualized with H₂O₂ and diaminobenzidine, and enhanced with nickel. To minimize variability, at least 5 sections per mice were analyzed under a bright-field DMRB RFY HC microscope (Leica). In each section, the percentage area occupied by β-amyloid, the density and the average size of β-amyloid accumulations were quantified using Image-J software (downloaded as a free software package from the public domain: http://rsb.info.nih.gov/ij/download.html).

**Results:** As STX64 showed a significant therapeutic effect in C. *elegans* neurodegeneration models, we tested the effect of this drug on cognitive alterations provoked by intrahippocampal β-amyloid oligomers infusion, an acute Alzheimer's disease (AD) mouse model (Figure 7A). Both, local and systemic STX64 treatments reverted cognitive deficiencies provoked by intrahippocampal administration of β-amyloid oligomers.

To evaluate the effect of STX64 oral treatment on amyloid pathology in a chronic AD mouse model, we assessed the effect of 3-4 weeks of STX64 oral administration on amyloid deposition in the neocortex (the cerebral cortex and the hippocampus) of >15-month-old APP-PS1 mice (Figure 7B). At the age of more than 15 months, which is also associated with a late stage of amyloid deposition in the neocortex of the APP-PS 1 model, analysis of β-amyloid immunoreactive area, plaque density and size revealed a significant reduction, except for plaque size in hippocampus, in mice treated with STX64 (Figure 7C-E). Interesting, when we compared β-amyloid deposition in old (>15 month-old) APP-PS1 mice treated with STX64 with the normal deposition rate of amyloid untreated APP-PS1 mice, we observed that STX64 reduced β-amyloid deposition in APP-PS1 mice older than 15 months to that observed in APP-PS1 mice of 10-12 months of age (Figure 7F).

All these results indicated that STX64 treatment in APP-PS1 mice reduce β-amyloid deposition. To assess whether the histological improvement correlated with amelioration of cognitive behavioural deficit, we compared the cognition capacity of APP-PS1 mice older than 15 month-old treated with vehicle or STX64 during 3-4 weeks. While vehicle-treated APP-PS1 mice showed a deficit in passive avoidance test (Figure 7G), those mice treated with STX64 completely reverted cognitive deficiencies, reaching similar levels to <15 month-old wild type mice. All these results point out that the alterations in β-amyloid metabolism provoked by STX64 reduce the cognitive behaviour deficiencies induced by β-amyloid accumulation in acute and chronic AD mouse models, indicating that it is plausible that STX64 and other sulfatase inhibitors could be used to treat proteopathies.

### Example 3: Effects of STX64 on a mouse model of Huntington's disease

**Mice strains and conditions:** The R6/1 mice (Yi Li et al., 2005. NeuroRX. 2(3): 447-464; Mangiarini et al., 1996. Cell. 87(3):493-506) used in this study were purchased from an authorized provider and they were habituated to standard animals housing conditions for 2-3 weeks (a 12 h light/dark cycle, temperature and humidity). Motor activity studies were performed with 2-month-old R6/1 mice. All experiments were performed in accordance with European Union guidelines (2010/63/EU) and with Spanish regulations for the use of laboratory animals in chronic experiments (RD 53/2013 on the care of experimental animals: BOE 08/02/2013), and the approval of the University Pablo de Olavide animal care committees was obtained prior to performing this study.

**Mice Oral STX administration:** STX64 was dissolved in drinking water at 0.005mg/ml. 1-month-old mice were exposed to STX solution for 1 month and the water intake was registered every day during the treatment. The estimated daily dose of STX64 was between 1-2 mg/Kg of body weight.

**Motor activity test:** Locomotor activity was tested in a non-stressful open-field (56x40x40cm) for 15 minutes. The distance traveled by the mice was estimated with Smart video-tracking software (Panlab).

**Results:** R6/1 mice exhibit a decrease in their activity at 2 months of age. This decrease in motor activity can be avoided by orally administering STX64 once they reach 1 month of age (see Figure 8).

This Example provides further evidence that the compositions of the present invention are suitable for treating protein-aggregation diseases.

### Example 4: Comparison of STX64 with EMATE in a C. elegans model of Alzheimer's disease (Reference example)

**Introduction:** In order to determine the effect of different inhibitors of steroid sulfatases (STS) in protein aggregation we assayed EMATE (CAS Number: 148672-09-7) in the strain GMC101, an improved model of AD in C. *elegans* (McColl et al., 2012. Mol Neurodegener. 7:57). EMATE, also named estrone sulfamate, is a potent irreversible inhibitor of estrone sulfatase (E1-STS) and dehydroepiandrosterone sulfatase (DHA-STS), but with strong estrogenic activity.

**Strain:** GMC101, genotype: dvIs100 [pCL354(unc-54:DA-A31-42) + pCL26(mtl-2: GFP)].

**Paralysis assay:** Worms were grown at a non-restrictive temperature of 16 °C until they reached the L4-young adult stage. The temperature was then shifted to 25 °C. The number of paralyzed and non-paralyzed animals were counted after an 18-hour incubation at the restrictive temperature of 25 °C.

**Statistical analysis:** The data were analyzed using a 2-tailed chi-square test in contingency table. Statistical analysis was performed using the GraphPad Prism software (version 7.00).

**Results:** We assayed the temperature-dependent phenotype of paralysis in different backgrounds and conditions. Absence of *sul-2* ameliorates the paralysis symptoms in GMC101 strain (see Figure 9A). Similar effects were seen for STX64 (see Figure 9B) and EMATE (see Figure 9C). Although, STX64 appeared to have the greatest effect.

## Claims

1. A composition comprising a steroid hormone sulfatase inhibitor for use in the treatment and/or prevention of a protein-aggregation disease, wherein amyloids and/or oligomers are removed, and/or their formation is prevented in the patient and/or animal, and wherein the formation of amyloids and/or oligomers is prevented in Parkinson's disease or Huntington's disease patients; or wherein the protein-aggregation disease is Parkinson's disease or Huntington's disease.

2. The composition for use according to claim 1, wherein the steroid hormone sulfatase inhibitor slows down the progression of a protein-aggregation disease by inhibiting the formation of protein aggregates and/or the sulfatase inhibitor delays the onset of a protein-aggregation disease by inhibiting the formation of protein aggregates.

3. The composition for use according to any one of claims 1-2, wherein the composition is a pharmaceutical composition comprising the steroid hormone sulfatase inhibitor according to any one of claims 1-2 and a pharmaceutically acceptable carrier and/or diluent.

4. The composition for use according to any one of claims 1-3, wherein the composition is administered orally.

5. The composition for use according to any one of claims 1-4, wherein a dose of 0.01 to 100 mg/kg is administered to the patient, preferably 0.01 to 10 mg/kg, more preferably, 0.05 to 1 mg/kg.

6. The composition for use according to any one of claims 1-5, wherein the composition is used in a combination therapy with donepezil, rivastigmine, galantamine, memantine, levodopa, carbidopa and/or tetrabenazine.

## Patentansprüche

1. Zusammensetzung umfassend einen Steroidhormon-Sulfatase-Hemmer für deren Verwendung bei der Behandlung und/oder Prävention von einer Proteinaggregationserkrankung, wobei Amyloide und/oder Oligomere beseitigt werden, und/oder deren Bildung im Patienten und/oder Tier vorgebeugt wird, und wobei die Bildung von Amyloiden und/oder Oligomeren in Patienten mit Parkinson-Krankheit oder Huntington-Krankheit vorgebeugt wird, oder wobei die Proteinaggregationserkrankung Parkinson-Krankheit oder Huntington-Krankheit ist.

2. Zusammensetzung für deren Verwendung nach Anspruch 1, wobei der Steroidhormon-Sulfatase-Hemmer das Fortschreiten einer Proteinaggregationserkrankung verlangsamt, indem die Bildung von Proteinaggregaten gehemmt wird, und/oder der Sulfatase-Hemmer den Ausbruch einer Proteinaggregationserkrankung verzögert, indem die Bildung von Proteinaggregaten gehemmt wird.

3. Zusammensetzung für deren Verwendung nach einem der Ansprüche 1-2, wobei die Zusammensetzung eine pharmazeutische Zusammensetzung ist, welche den Steroidhormon-Sulfatase-Hemmer nach einem der Ansprüche 1-2 und einen pharmazeutisch akzeptablen Träger und/oder Verdünnungsmittel umfasst.

4. Zusammensetzung für deren Verwendung nach einem der Ansprüche 1-3, wobei die Zusammensetzung oral verabreicht wird.

5. Zusammensetzung für deren Verwendung nach einem der Ansprüche 1-4, wobei dem Patienten eine Dosis von 0,01 bis 100 mg/kg, vorzugsweise 0,01 bis 10 mg/kg, weiter vorzugsweise 0,05 bis 1 mg/kg, verabreicht wird.

6. Zusammensetzung für deren Verwendung nach einem der Ansprüche 1-5, wobei die Zusammensetzung in einer Kombinationstherapie mit Donepezil, Rivastigmin, Galantamin, Memantin, Levodopa, Carbidopa und/oder Tetrabenazin verwendet wird.

## Revendications

1. Composition comprenant un inhibiteur de l'hormone stéroïdienne sulfatase pour son utilisation dans le traitement et/ou la prévention d'une maladie d'agrégation des protéines, dans laquelle les amyloïdes et/ou les oligomères sont éliminés, et/ou leur formation est empêchée chez le patient et/ou l'animal, et dans laquelle la formation d'amyloïdes et/ou d'oligomères est empêchée chez des patients atteints de la maladie de Parkinson ou de la maladie de Huntington ; ou dans laquelle la maladie d'agrégation des protéines est la maladie de Parkinson ou la maladie de Huntington.

2. Composition pour son utilisation selon la revendication 1, dans laquelle l'inhibiteur de l'hormone stéroïdienne sulfatase ralentit la progression d'une maladie d'agrégation des protéines par le biais de l'inhibition de la formation d'agrégats de protéines et/ou l'inhibiteur de sulfatase retarde l'apparition d'une maladie d'agrégation des protéines par le biais de l'inhibition de la formation d'agrégats de protéines.

3. Composition pour son utilisation selon l'une quelconque des revendications 1-2, dans laquelle la composition est une composition pharmaceutique comprenant l'inhibiteur de l'hormone stéroïdienne sulfatase selon l'une quelconque des revendications 1-2 et un véhicule et/ou diluent pharmaceutiquement acceptable.

4. Composition pour son utilisation selon l'une quelconque des revendications 1-3, dans laquelle la composition est administrée par voie orale.

5. Composition pour son utilisation selon l'une quelconque des revendications 1-4, dans laquelle une dose de 0,01 à 100 mg/kg est administrée au patient, de préférence de 0,01 à 10 mg/kg, plus de préférence de 0,05 à 1 mg/kg.

6. Composition pour son utilisation selon l'une quelconque des revendications 1-5, dans laquelle la composition est utilisée dans une thérapie de combinaison avec le donépézil, la rivastigmine, la galantamine, la mémantine, le lévodopa, le carbidopa et/ou la tétrabénazine.
